# EUROPEAN PATENT SPECIFICATION

(11) **EP 3 721 936 B1**
(45) Date of publication and mention of the grant of the patent: **12.10.2022**
(21) Application number: 19167946.3
(22) Date of filing: 08.04.2019
(51) Int. Cl.: A61N 1/05, A61N 1/36, A61N 1/375

(54) **ELECTRODE ARRAY AND BIOARTIFICIAL IMPLANT SYSTEM INCLUDING THE SAME**
ELEKTRODENANORDNUNG UND BIOARTIFIZIELLES IMPLANTATSYSTEM DAMIT
RÉSEAU D'ÉLECTRODES ET SYSTÈME D'IMPLANT BIOARTIFICIEL LE COMPRENANT

(43) Date of publication of application: 14.10.2020
(73) Proprietor: Todoc Co., Ltd., Seoul 08826 (KR)
(72) Inventor: MIN, Kyou Sik, 16493 Gyeonggi-do (KR); KIM, Jin Won, 07980 Seoul (KR)
(74) Representative: Davies, Gregory Mark

(56) References cited:
- EP-A1- 3 386 214
- WO-A1-03/003791
- WO-A1-2015/030734

## Description

### BACKGROUND

### 1. Field of the Invention

Exemplary embodiments relate to an electrode array and a bioartificial implant system including the same.

### 2. Discussion of Related Art

Many medical devices have been developed to help people who have lost a specific function due to either a congenital or acquired cause. As such a medical device, bioartificial implant systems including nerve assist devices have also been developed.

A cochlear implant device, which is one of the bioartificial implant systems that helps people whose auditory nerve function is intact to hear a sound by stimulating the auditory nerve with electricity, is perceived as the most effective device among the nerve assist devices developed so far. Transplant operations of the cochlear implant device are rising every year.

The cochlear implant device may include an external device provided outside the body part and an internal device provided inside the body part.

The external device serves to receive a sound from the outside of the human body and convert the received sound into an electrical signal. The external device may include a microphone (voice transmitter), a speech processor (language synthesizer), and a transmission antenna (transmitter). In this case, the microphone and the transmission antenna may be configured by being combined with a headset.

The internal device serves to stimulate the auditory nerve in response to a signal transmitted from the external device. The internal device may include a voice receiver and an electrode for reception and stimulation.

The cochlear implant device may convert a sound signal transmitted to the microphone attached to an exterior of the human body into an electrical signal for physical vibration through amplification and filtration in a speech processor without a tympanic membrane or auditory ossicles, thereby transmitting the converted electrical signal to an auditory nerve fiber through an electrode implanted in a cochlea.

However, since a conventional electrode is manufactured so that a platinum electrode and a wire are manually aligned and silicon-molded, there is a problem in that a cost and a time are increased and a yield is lowered. Further, since an area of the conventional electrode is small, there is a problem in that a current stimulus is relatively small.

EP3386214, WO2015030734 and WO2003003791 may be described as disclosing an electrode array including a body including a first groove extending in a first direction, a substrate disposed on the first groove, a plurality of lines disposed on one surface of the substrate, a plurality of electrodes disposed to be spaced apart from each other on the substrate in the first direction, and a mold member configured to fix the plurality of electrodes to the body, wherein each of the plurality of electrodes includes a stimulation portion exposed to an outer side of the mold member and a first end portion electrically connecting the stimulation portion to the plurality of lines.

### SUMMARY OF THE INVENTION

The exemplary embodiments are directed to an electrode array which is easily manufactured, and a bioartificial implant system including the same.

Further, the exemplary embodiments are directed to an electrode array having an electrode with a wide area so as to be able to apply a relatively large current stimulus, and bioartificial implant system including the same.

According to the invention there is provided an electrode array in accordance with claim 1.

Each of the plurality of electrodes may include a connecting portion bent upward from the first end portion and include the stimulation portion disposed to face the substrate from the connecting portion.

A deformed portion by being partially heated may be included between the connecting portion and the first end portion and between the connecting portion and the stimulation portion.

The deformed portion may be formed to extend in the first direction.

Each of the plurality of electrodes may include a second end portion bent downward from the stimulation portion toward the substrate.

Each of the substrate and the cover may have a first cross-sectional area, in which the plurality of electrodes are disposed, that is greater than a second cross-sectional area between the plurality of electrodes.

Each of the plurality of electrodes may include platinum (Pt) and iridium (Ir).

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 is a conceptual diagram of a bioartificial implant system according to one non-claimed embodiment of the present disclosure;
FIG. 2 is an exemplary diagram illustrating a case in which a second unit of the bioartificial implant system according to one non-claimed embodiment of the present disclosure is applied to a human body;
FIG. 3 is a conceptual diagram of the second unit according to one non-claimed embodiment of the present disclosure;
FIG. 4 is a conceptual diagram of an electrode array according to one non-claimed embodiment of the present disclosure;
FIG. 5 is a diagram illustrating an arrangement of a plurality of electrodes of FIG. 4;
FIG. 6 is a cross-sectional view taken along line A-A of FIG. 4;
FIG. 7 is a diagram illustrating the electrodes formed on a substrate;
FIG. 8 is a diagram illustrating a cover formed on the substrate on which the electrodes are formed;
FIG. 9 is a diagram illustrating a case in which the substrate and the cover are partially incised to expose the electrodes;
FIG. 10 is a diagram illustrating a process of irradiating a laser in a direction perpendicular to an extension direction of the substrate;
FIG. 11 is a diagram illustrating a state in which the electrode is bent by laser irradiation;
FIG. 12 is a diagram illustrating a process of manufacturing a body;
FIG. 13 is a diagram illustrating a state in which a first groove is formed in the body;
FIG. 14 is a diagram illustrating a process of disposing the electrodes in the body;
FIG. 15 is a diagram of a process of fixing the electrodes to the body with a mold member;
FIG. 16 is a conceptual diagram of an electrode array according to an embodiment of the claimed invention;
FIG. 17 is a cross-sectional view taken along line B-B of FIG. 16;
FIG. 18 is a cross-sectional view taken along line C-C of FIG. 16;
FIG. 19 is a diagram illustrating a process of bending an exposed electrode;
FIG. 20 is a diagram illustrating a process of disposing electrodes on a manufactured body;
FIG. 21 is a diagram illustrating a process of filling a mold member to fix the electrodes to the body;
FIG. 22 is a conceptual diagram of an electrode array according to still another non-claimed embodiment of the present disclosure;
FIG. 23 is a diagram illustrating an arrangement of a plurality of electrodes of FIG. 22;
FIG. 24 is a cross-sectional view taken along line D-D of FIG. 22; and
FIG. 25 is a cross-sectional view taken along line E-E of FIG. 22.

### DETAILED DESCRIPTION OF EXEMPLARY EMBODIMENTS

Hereinafter, the present invention will be described with reference to the accompanying drawings. However, the present invention may be implemented in various different forms, and thus it is not limited to exemplary embodiments which will be described herein. In the drawings, some portions not related to the description will be omitted in order to clearly describe the present invention, and similar reference numerals are given to similar components throughout this disclosure.

Throughout this disclosure, when a portion is referred to as being "connected to" another portion, this includes not only "being directly connected to" but also "being indirectly connected to" by interposing another member between the portion and another portion. Also, when a portion is referred to as "including" a component, it may mean that another component is further included and is not to be excluded unless specifically stated otherwise.

In addition, the following exemplary embodiments are illustrative, and thus the scope of the present invention is not limited thereto, and configurations of the exemplary embodiments may be combined with each other to constitute a new exemplary embodiment. Further, it should be understood that the detailed configurations which can be easily substituted and/or modified by those skilled in the art although not described in detail in this disclosure can be applicable to the exemplary embodiments of the present invention, and also details of the exemplary embodiments of the present invention are illustrative.

Hereinafter, exemplary embodiments of the present invention will be described in detail with reference to the accompanying drawings.

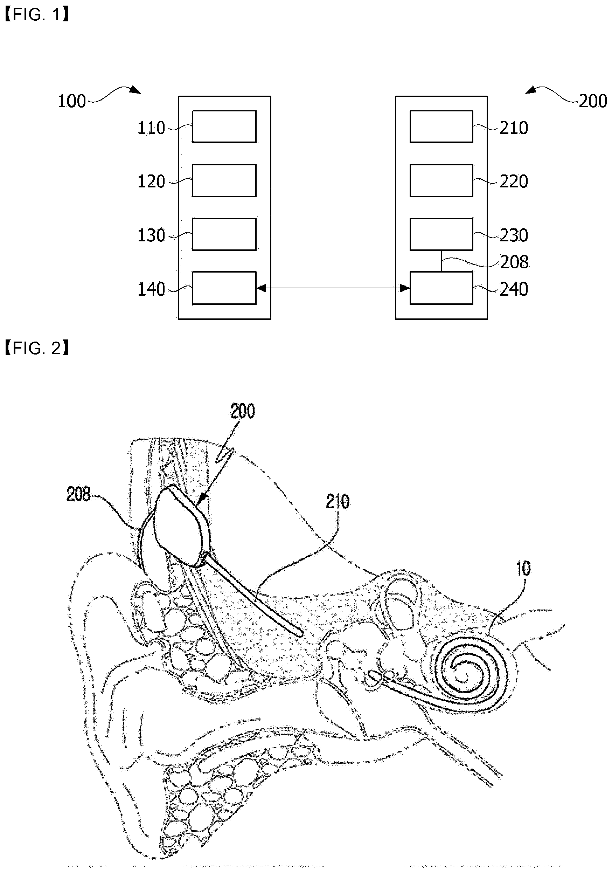

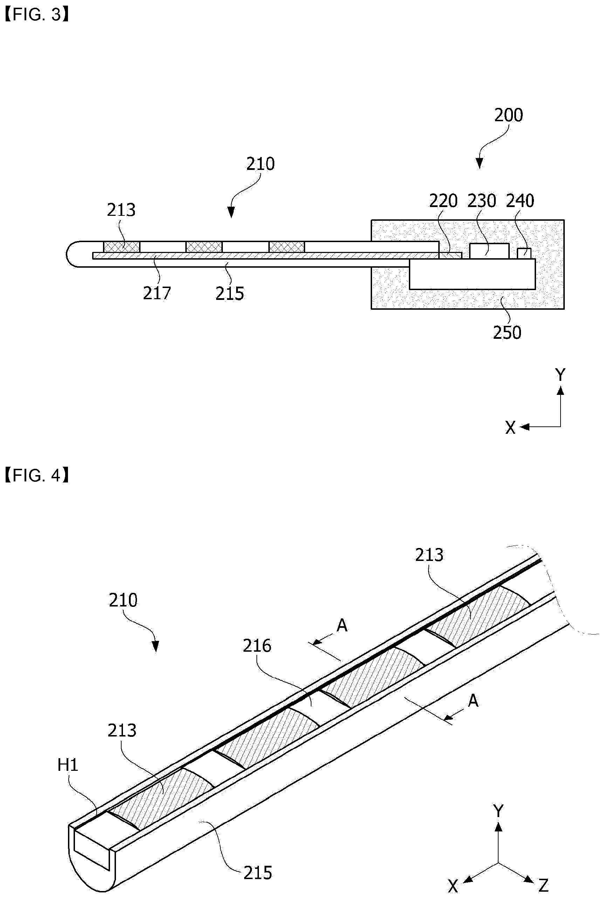

FIG. 1 is a conceptual diagram of a bioartificial implant system according to one non-claimed embodiment of the present disclosure, FIG. 2 is an exemplary diagram illustrating a case in which a second unit of the bioartificial implant system according to one non-claimed embodiment of the present disclosure is applied to a human body, and FIG. 3 is a conceptual diagram of the second unit according to one embodiment of the present disclosure;

Referring to FIG. 1, a bioartificial implant system according to an embodiment may include a first unit 100 and a second unit 200 which are communicatable with each other. Hereinafter, an example of a cochlear implant system, which is one of bioartificial implant systems, will be described. However, the present embodiment is not limited thereto. For example, the second unit 200 may be a device for providing an electrical signal to other parts or organs of the human body.

The first unit 100 may convert a sound signal into an electrical signal to provide the converted electrical signal and may include a first coil 140 provided outside a person's skin and configured to supply electric power. The first unit 100 may be disposed outside the skin. That is, the first unit 100 may be installed outside the human body instead of being implanted in the human body.

The first unit 100 may include a voice transmitter 110, a voice processor 120, a transmitter 130, and the first coil 140.

The voice transmitter 110 may detect an external sound signal. The sound signal may include a voice signal or an acoustic signal. Various electronic devices capable of detecting a sound signal may be selected as the voice transmitter 110.

The voice processor 120 may receive the sound signal detected by the voice transmitter 110 and convert the received sound signal into an electrical signal. The voice processor 120 may include a speech processor.

The transmitter 130 may receive the electrical signal from the voice processor 120 and transmit the received electrical signal. The first coil 140 may supply electric power. However, the present invention is not particularly limited thereto, and the transmitter 130 may be omitted. That is, the first unit 100 may not include a separate transmitter 130. In this case, the first unit 100 may receive the electrical signal from the voice processor 120 and transmit the received electrical signal to the second unit 200 through the first coil 140 along with electrical power transmission.

The first unit 100 may include a power supplier (not shown). The power supplier is a configuration for supplying electric power to the first unit 100. The power supplier may include a replaceable battery or a rechargeable battery.

The power supplier may receive electric power from the outside and store the electric power therein. For example, the power supplier may include a capacitive element such as a capacitor. The capacitive element may receive electric power through wires from an external power supplier and store the received electric power therein. Alternatively, the capacitive element may receive electric power from an external power supplier through the first coil 140 of the first unit 100 in a wireless manner.

For example, the first coil 140 may receive wirelessly electric power from a coil of the external power supplier through electromagnetic induction in a charging mode and store the received electric power in the capacitive element. In a transmission mode, the first coil 140 may transmit the electric power stored in the capacitive element to a second coil 240 of the second unit 200 in a wireless manner.

In this case, the transmission of electric power through the coil may use an electromagnetic induction phenomenon, but the present invention is not limited thereto, and other types of wireless power transmission techniques may be used.

The second unit 200 may be inserted into the skin. For example, the second unit 200 may be inserted into the subcutaneous fat layer or may be implanted into the human body.

The second unit 200 may receive the electrical signal from the first unit 100 to stimulate an auditory nerve fiber in a cochlea 10.

The second unit 200 may include a receiver 230, a circuit part 220 for processing a signal received from the first unit 100 to generate a stimulation signal, and an electrode array 210 having a plurality of electrodes (not shown) for stimulating the auditory nerve fiber in the cochlea 10 according to the stimulation signal transmitted from the circuit part 220.

The receiver 230 may receive a signal from the first unit 100. For example, the receiver 230 may include the second coil 240 for receiving a signal with electric power from the first coil 140. When the first coil 140 of the first unit 100 transmits the electric power, the first coil 140 may transmit a data signal for electrical stimulation together with an electric power signal. For example, the first coil 140 may transmit the electric power together with the data signal by varying an amplitude or a phase of the electric power signal.

Alternatively, the receiver 230 may directly receive a signal from the transmitter 130 of the first unit 100. The transmitter 130 and the receiver 230 may communicate with each other through various communication technologies which are not limited to specific communication technology. Alternatively, it is also possible to communicate the data signal separately from the electric power signal via a separate communication means or a separate frequency that is not described above.

The circuit part 220 may process the signal received at the receiver 230 to generate a stimulation signal. The circuit part 220 may include an integrated circuit (IC) for generating a stimulation signal.

The electrode array 210 may have a structure in which a plurality of electrodes (not shown) are disposed on an insulating layer. The electrode array 210 may be formed to be thin enough to be inserted into the cochlea 10 of the human body.

Referring to FIG. 3, the receiver 230 and the circuit part 220 of the second unit 200 may be disposed on a support substrate and may be accommodated in a housing 250. The housing 250 may be made of a material that is identical to a polymer material of the support substrate, but the present invention is not particularly limited thereto.

The electrode array 210 may be formed to extend in a first direction (X-axis direction). An extension length of the electrode array 210 is not particularly limited. The electrode array 210 may have a predetermined length which is inserted into the cochlea 10 of the human body so as to be able to make stimulation.

A plurality of electrodes 213 may be applied with a current signal transmitted from the circuit part 220 to stimulate an auditory nerve fiber in the cochlea 10. Further, the plurality of electrodes 213 may collect, detect, and record a biological signal from the auditory nerve fiber.

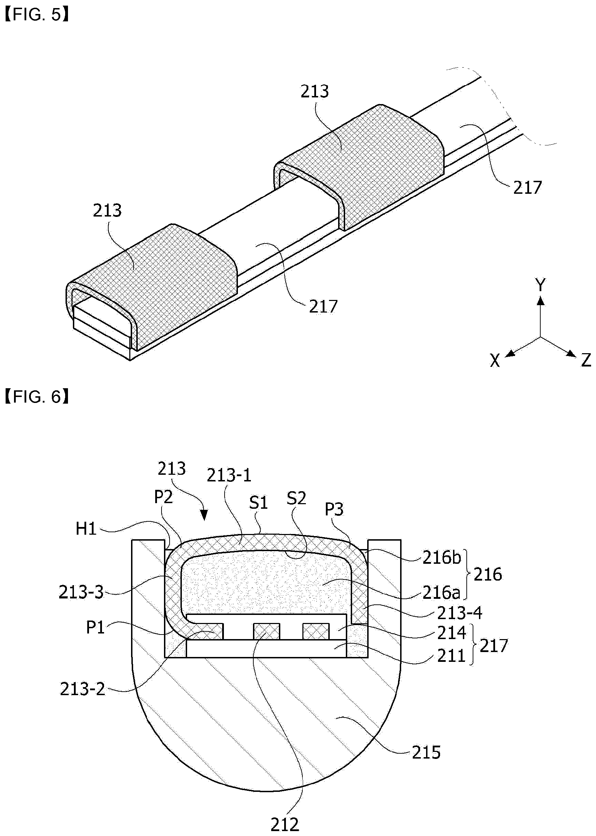

FIG. 4 is a conceptual diagram of an electrode array according to one embodiment of the present invention, FIG. 5 is a diagram illustrating an arrangement of a plurality of electrodes of FIG. 4, and FIG. 6 is a cross-sectional view taken along line A-A of FIG. 4;

Referring to FIGS. 4 to 6, the electrode array 210 according to the present embodiment includes a body 215 having a first groove H1 extending in the first direction (X-axis direction), a substrate 211 disposed in the first groove H1, a plurality of lines 212 disposed on one surface of the substrate 211, the plurality of electrodes 213 disposed to be spaced apart from each other on the substrate 211 in the first direction, and a mold member 216 for fixing the plurality of electrodes 213 to the body 215.

The electrode array 210 may be formed to extend in the first direction (X-axis direction). A length of the electrode array 210 is not particularly limited. The electrode array 210 may be formed to be thin enough to be inserted into the cochlea 10 of the human body and formed so as to be able to make stimulation.

The body 215 may form an appearance of the electrode array 210 and accommodate the plurality of electrodes 213. The substrate 211 included in the body 215 may be made of a polymer material, but present invention is not limited thereto. For example, the body 215 may be made of a liquid crystal polymer (LCP) material. In addition to the LCP material, various polymers may be selected as a material of the body 215. For example, a polymer such as parylene, a cyclo olefin polymer (COP), a cyclic olefin copolymer (COC), or the like may be selected as a material of the substrate 211.

The plurality of electrodes 213 may be disposed to be spaced apart from each other in the first direction. Areas of and intervals between the plurality of electrodes 213 are not particularly limited. The plurality of electrodes 213 may be fixed to the body 215 by the mold member 216.

Referring to FIGS. 5 and 6, the substrate 211 may be disposed in the first groove H1. The substrate 211 may be made of an LCP material, but the present invention is not particularly limited thereto. The substrate 211 may employ any material as long as it can be easily patterned and is flexible.

A cover 214 may be disposed on the substrate 211. The cover 214 may be made of a material that is identical to that of the substrate 211. The plurality of lines 212 and the plurality of electrodes 213 may be stacked between the substrate 211 and the cover 214. A conventional lamination method may be applied to both of the plurality of lines 212 and the plurality of electrodes 213.

The mold member 216 may fill in the first groove H1 to fix the substrate 211 and the electrodes 213 to the first groove H1. The mold member 216 may be made of a material that is identical to that of the body 215.

The plurality of electrodes 213 may be respectively electrically connected to the plurality of lines 212. Thus, a current may be selectively applied to the plurality of electrodes 213.

The plurality of electrodes 213 may extend from a side surface of the substrate 211 to be bent to face one surface of the substrate 211. Therefore, an area of the electrode 213 may become larger as compared to a structure in which the electrode 213 is formed on the substrate 211.

The plurality of electrodes 213 may include a stimulation portion 213-1 exposed to an outer side of the mold member 216, a first end portion 213-2 for electrically connecting the stimulation portion 213-1 to the line 212, and a connecting portion 213-3 bent upward from the first end portion 213-2.

The stimulation portion 213-1 may be defined as a position which is exposed to the outside of the mold member 216 and from which electrical stimulation is able to be applied. According to the present embodiment, the electrode 213 may be disposed to be bent about the substrate 211 and an upper end portion of the electrode 213 may be exposed from the mold member 216. With the above-described configuration, there is an advantage in that the electrode 213 having a large area may be exposed to the outside while the substrate 211 may be stably fixed in the body 215.

One surface S2 of the stimulation portion 213-1 may be fixed by the mold member 216 and may disposed to face the substrate 211. Further, the other surface S1 may be exposed to the outside to transmit stimulation to the human body when an electrical signal is applied.

The first end portion 213-2 may be electrically connected to the line 212 disposed on the substrate 211, and the connecting portion 213-3 may be bent upward for the first end portion 213-2 to form a space between the stimulation portion 213-1 and the substrate 211. The mold member 216 may be disposed in the space between the stimulation portion 213-1 and the substrate 211.

A second end portion 213-4 may be bent downward from the stimulation portion 213-1 toward the substrate 211. Accordingly, the second end portion 213-4 may be fixed to an interior of the mold member 216. Thus, the first end portion 213-2 and the second end portion 213-4 may be stably fixed to the interior of the mold member 216.

The mold member 216 may include a first mold part 216a disposed inside a bent electrode 213 and a second mold part 216b disposed between an exterior of the electrode 213 and the first groove H1. The first mold part 216a may be connected to the second mold part 216b. With the above-described configuration, the mold member 216 may be uniformly disposed in an interior and the exterior of the electrode 213 to be stably fixed.

The plurality of electrodes 213 may each include deformed portions P1, P2, and P3 which are made of being partially heated. The deformed portions P1, P2, and P3 may be disposed at regions at which the electrode 213 is bent. For example, the deformed portions P2 and P3 may be disposed between the connecting portion 213-3 and the stimulation portion 213-1 and between the stimulation portion 213-1 and the second end portion 213-4.

The deformed portions P1, P2, and P3 may be heated by laser irradiation to cause local bending of the electrode 213. In this case, the deformed portions P1, P2, and P3 may be formed to extend in the first direction.

However, the present invention is not limited thereto, and the deformed portions P1, P2, and P3 may be deformed due to different causes. For example, the first deformed portion P1 and the third deformed portion P3 may be partially heated by laser irradiation to cause bending, whereas the second deformed portion P2 may be bent by an external physical force. Thus, metal structures in the first and third deformed portions P1 and P3 may be different from a metal structure of the second deformed portion P2.

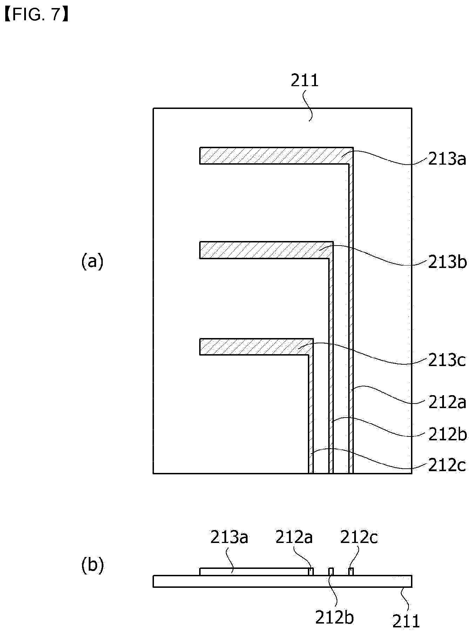

FIGS. 7 to 15 are diagrams for describing a method of manufacturing an electrode array according to one non-claimed embodiment of the present disclosure.

Referring to FIG. 7, after an electrode layer is entirely formed on the substrate 211, the plurality of lines 212 and the plurality of electrodes 213 may be patterned. A patterning method is not particularly limited. All conventional patterning methods used in a semiconductor manufacturing process may be applied. For example, patterning may be performed by selective etching using a mask.

The substrate 211 may be made of an LCP material, but the present invention is not particularly limited thereto. The substrate 211 may employ any material as long as it can be easily patterned and is flexible.

For example, the first electrode 213a may be connected to a first line 212a, a second electrode 213b may be connected to a second line 212b, and a third electrode 213c may be connected to a third line 212c. Consequently, the first electrode 213a to the third electrode 213c may be separately driven.

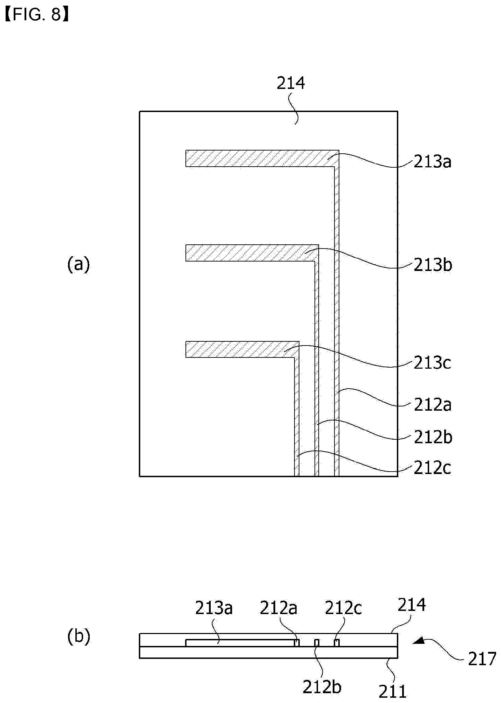

Referring to FIG. 8, the cover 214 may be formed on the substrate 211. The cover 214 may be made of a material that is identical to that of the substrate 211. The plurality of lines 212 and the plurality of electrodes 213 may be stacked between the substrate 211 and the cover 214. A conventional lamination method may be applied to both of the plurality of lines 212 and the plurality of electrodes 213.

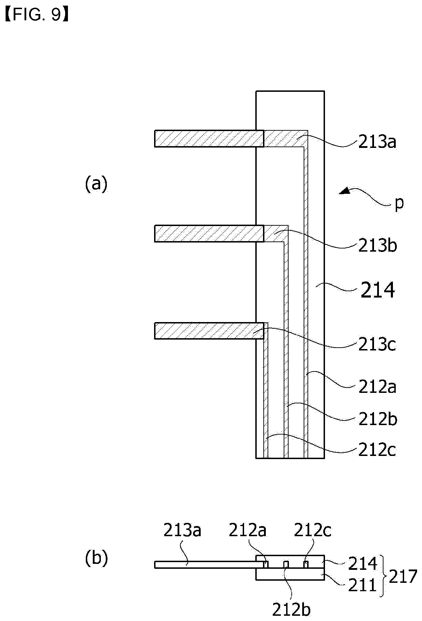

Referring to FIG. 9, some portion of the cover 214 and the substrate 211 may be incised to expose portions of the plurality of electrodes 213. The cover 214 and the substrate 211 may be removed using laser scribing or etching. For example, the cover 214 and substrate 211 may be removed using laser scribing. In this case, a surface of the electrode 213 should not be reacted with a laser beam so that an irradiation time of a laser may be controlled to be very short. However, a method of removing the cover 214 and the substrate 211 is not particularly limited thereto. In this case, the plurality of electrodes 213 may be defined as regions exposed to the outer side of the substrate 211. Thus, the plurality of electrodes 213 may have the same area.

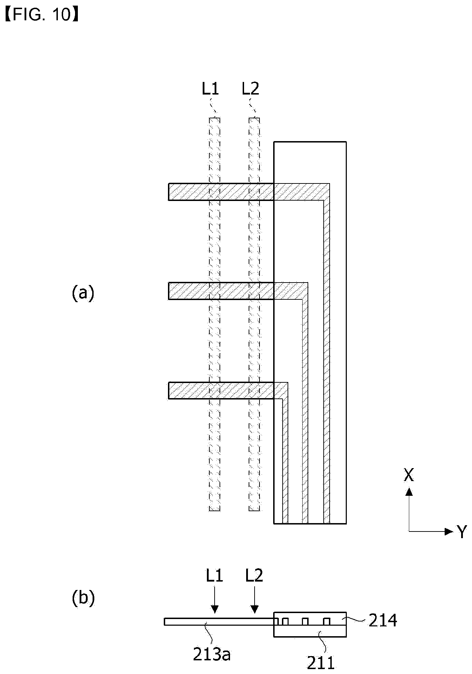

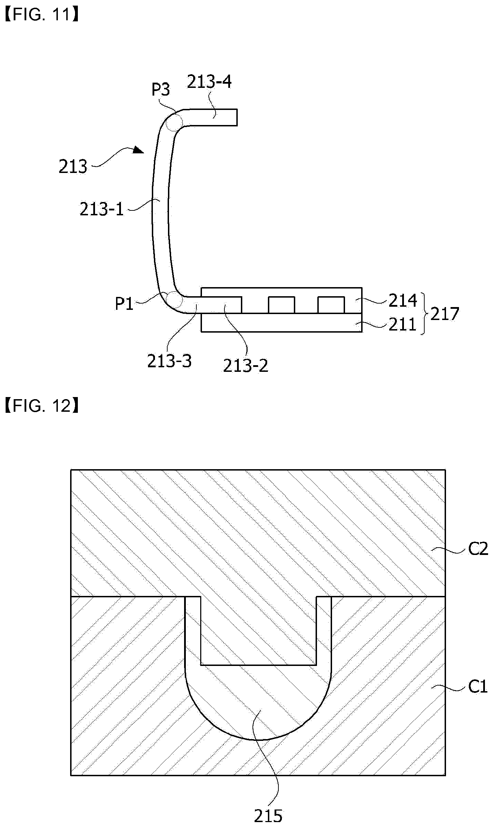

Referring to FIGS. 10 and 11, the plurality of electrodes 213 may be irradiated with a laser to cause bending by partially heating the plurality of electrodes 213. When a concentration of the laser is high, the electrode 213 may be separated due to high energy so that an output of the laser may be lowered using a defocusing lens or the like.

The output of the laser may be in the range of 0.4 W to 10 W. When the output is 0.4 W or less, the electrode 213 is not heated so that bending does not occur, whereas when the output is 10 W or more, an intensity of the laser is too high so that the electrode 213 may be cut.

The laser may be irradiated in the first direction (X-axis direction). Accordingly, the deformed portions P1 and P3 formed on the plurality of electrodes 213 may be formed in the extension direction of the substrate 211. Further, the laser may be irradiated with the number of times L1 and L2 in a second direction (Y-axis direction) perpendicular to the extension direction of the substrate 211.

Referring to FIG. 11, the electrode 213 may be bent due to the plurality of deformed portions P1 and P3. According to the present embodiment, since the electrode 213 is bent when the laser is irradiated, there may be an advantage of simplifying a process of manually bending the plurality of electrodes 213.

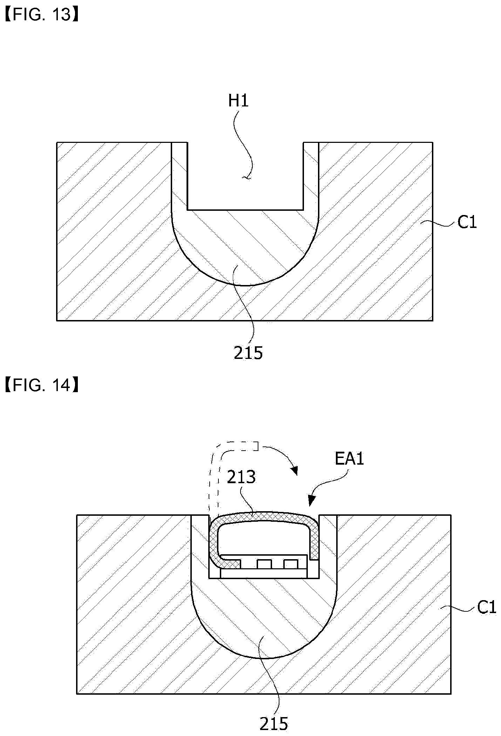

Referring to FIGS. 12 and 13, the body 215 may be formed by injecting a mold resin between a first cast C1 and a second cast C2. In this case, the first groove H1 may be formed in the body 215.

Referring to FIG. 14, after the substrate 211 and the plurality of electrodes 213 are disposed in the first groove H1, the plurality of electrodes 213 may be inserted into the first groove H1. When a portion of the electrode 213 is not inserted into the first groove H1, the electrode 213 may be inserted into the first groove H1 by a manual operation or a machine.

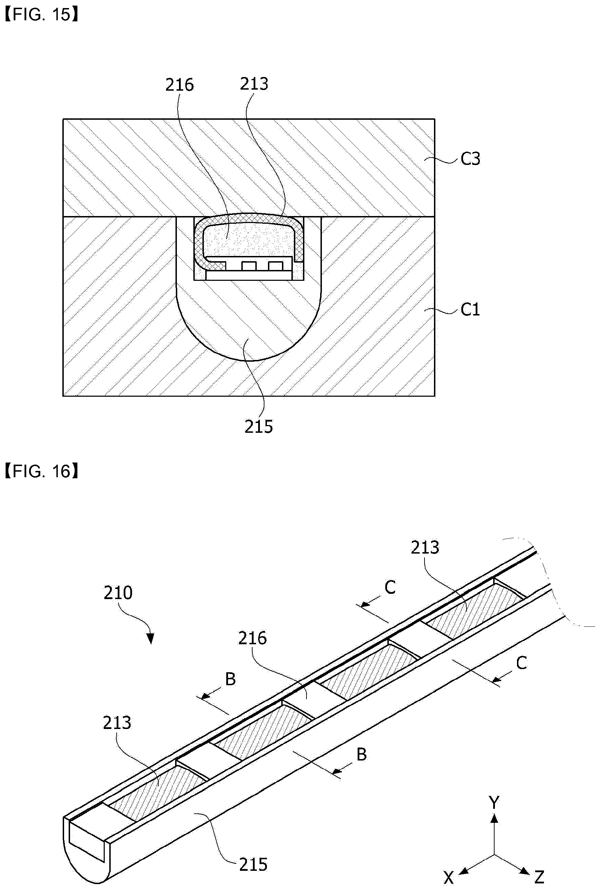

Referring to FIG. 15, the mold member 216 may be formed by covering the first cast C1 with a third cast C3 and injecting a mold resin therebetween. In this case, the electrode 213 is bent such that the mold resin may be injected into an inner space formed by the electrode 213 being bent.

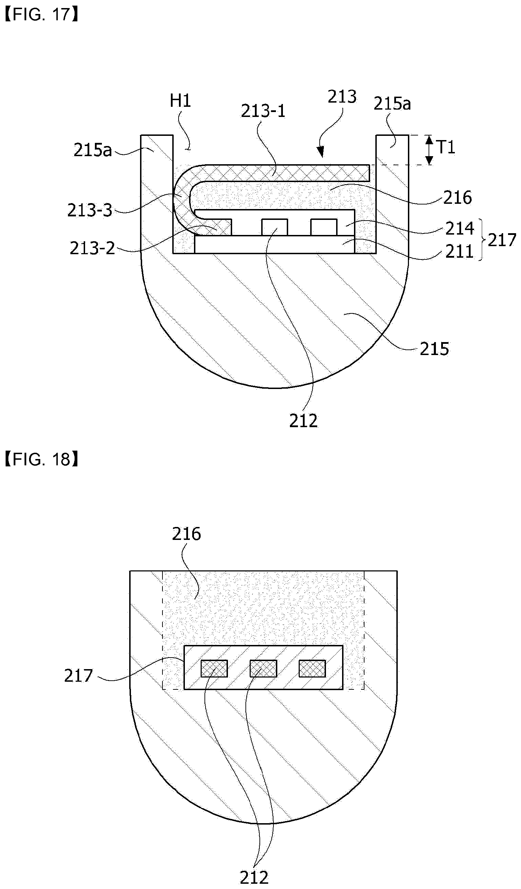

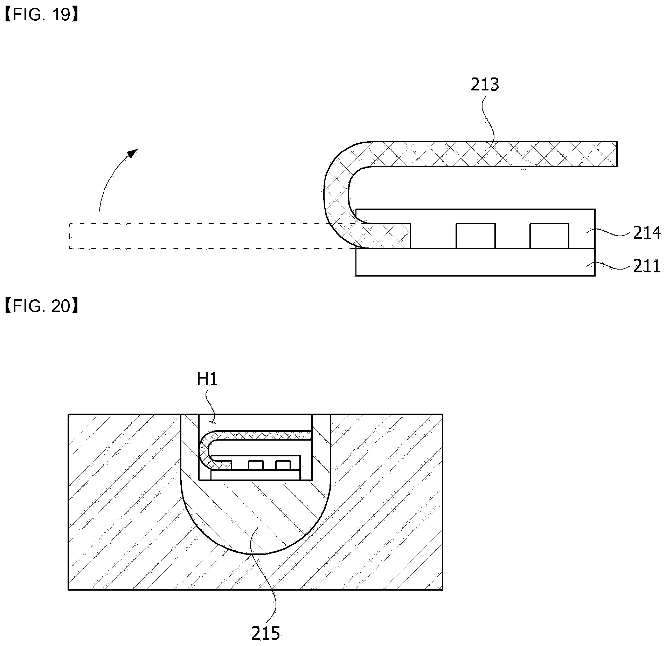

FIG. 16 is a conceptual diagram of an electrode array according to the claimed invention, FIG. 17 is a cross-sectional view taken along line B-B of FIG. 16, FIG. 18 is a cross-sectional view taken along line C-C of FIG. 16, and FIG. 19 is a diagram illustrating a process of bending an exposed electrode.

Referring to FIGS. 16 to 18, the electrode 213 includes a stimulation portion 213-1 exposed to the outside of the mold member 216 and include a first end portion 213-2 connecting the stimulation portion 213-1 to the line 212 of the substrate 211.

According to the present embodiment, the electrode 213 may be exposed on the side surface of the substrate 211 to be bent to face one surface of the substrate 211. According to the present embodiment, it is possible to maximally bend the electrode 213 toward the substrate 211 without applying bending due to laser irradiation. Therefore, the stimulation portion 213-1 of the electrode 213 may be disposed lower than the first groove H1. A gap T1 between an upper surface of the first groove H1 and the stimulation portion 213-1 may be in the range of 10 µm to 20 µm. With the above-described configuration, as shown in FIG. 18, it is possible to secure a thickness of the mold member 216 formed on the substrate 211 to stably fix the substrate 211. With the above-described structure, there is an advantage of simplifying the process of bending the electrode 213.

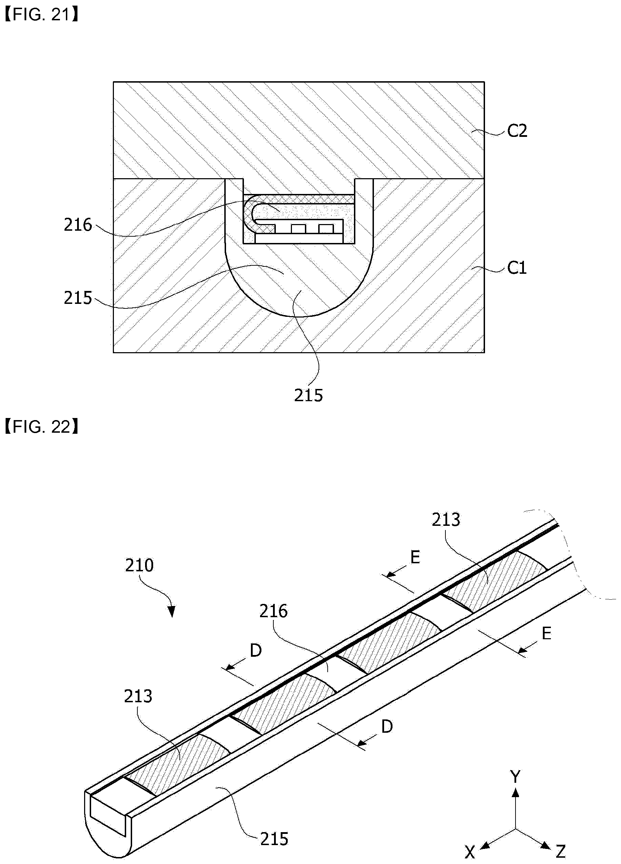

Referring to FIG. 19, a method of exposing the electrode 213 by incising the substrate 211 and the cover 214 may be identical to the method described with reference to FIGS. 7 to 9. Thereafter, the exposed electrode 213 may be bent toward the substrate 211. In this case, as described above, the laser may be defocused to bend the electrode 213. Alternatively, the electrode 213 may be bent by a machine or a manual operation. Referring to FIG. 20, the substrate 211 and the electrode 213 may be disposed on a body 215, and then a mold resin may be injected as shown in FIG. 21 to manufacture the electrode array 210.

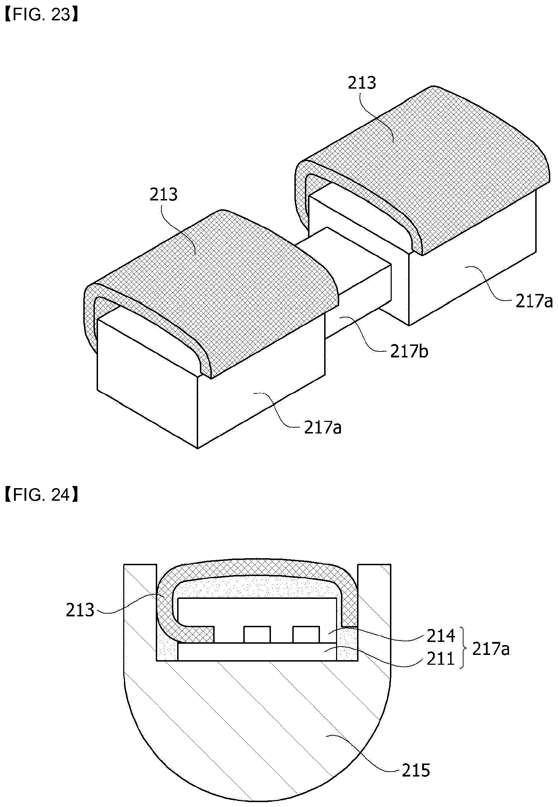

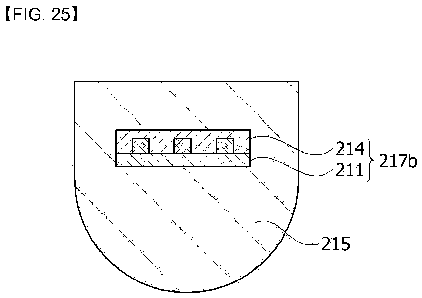

FIG. 22 is a conceptual diagram of an electrode array according to still another non-claimed embodiment of the present disclosure, FIG. 23 is a diagram illustrating an arrangement of a plurality of electrodes of FIG. 22, FIG. 24 is a cross-sectional view taken along line D-D of FIG. 22, and FIG. 25 is a cross-sectional view taken along line E-E of FIG. 22.

Referring to FIGS. 22 to 24, the substrate 211 and the cover 214 may include a first portion 217a in which the plurality of electrodes 213 are disposed, and a second portion 217b in which the plurality of electrodes 213 are not disposed. A first cross-sectional area of the first portion 217a may be greater than a second cross-sectional area of the second portion 217b. The first cross-sectional area and the second cross-sectional area may be adjusted using a semiconductor pattern process.

With the above-described configuration, as shown in FIG. 24, there may be an advantage in that the first portion 217a, in which the plurality of electrodes 213 are disposed, is manufactured to be relatively thicker and is brought into close contact with a first groove, whereas as shown in FIG. 25, the second portion 217b disposed between the first portions 217a is stably fixed by being completely surrounded by the mold member 216.

With the above-described structure, since a portion at which the electrode 213 is not formed is manufactured to be relatively thin, there may be an advantage in that the second portion 217b not having the electrode 213 is sufficiently covered even though a mold member 216 is only formed to completely enclose a bent position of the electrode 213.

According to the exemplary embodiments, it is possible to easily manufacture an electrode array by injecting a mold member into an electrode.

Further, an area of an electrode is widened such that a relatively large current stimulus can be applied.

It should be understood that effects of the present invention are not limited to the above-described effect and include all effects that can be deduced from the detailed description of the present invention or the configuration thereof defined by the appended claims.

The above-described description of the present invention is intended only for an illustrative purpose, and it can be easily understood that other concrete forms can be devised by those skilled in the art without departing from the scope as defined in the appended claims.

## Claims

1. An electrode array comprising:
a body (215) including a first groove (H1) extending in a first direction (X);
a substrate (211) disposed on the first groove (H1);
a plurality of lines (212) disposed on one surface of the substrate (211);
a plurality of electrodes (213) disposed to be spaced apart from each other on the substrate (211) in the first direction, each of the plurality of electrodes (213) including a stimulation portion (213-1), a first end portion (213-2), and a connecting portion (213-3) connecting the stimulation portion (213-1) to the first end potion (213-2), and the first end potion (213-2) being electrically connected to each of the plurality of lines (212);
a cover (214) disposed on the substrate (211) and configured to cover the first end portion (213-2) of each of the plurality of electrodes (213); and
a mold member (216) configured to fix the plurality of electrodes (213) to the body (215),
wherein the stimulation portion (213-1) is exposed to an outer side of the mold member, and the first end portion (213-2) electrically connecting the stimulation portion (213-1) to the plurality of lines (212) via the connection portion (213-3),
wherein the stimulation portion (213-1) is disposed lower than the upper surface of the first groove (H1), and
wherein some portion of the cover (214) and the substrate (211) incised to expose portions of the plurality of electrodes (213).

2. The electrode array of claim 1, wherein the connecting portion (213-3) is bent upward from the first end portion (213-2) and the stimulation portion (213-1) is disposed to face the substrate (211) from the connecting portion (213-3).

3. The electrode array of claim 2, wherein deformed portions (P1, P2) by being partially heated are included between the connecting portion (213-3) and the first end portion (213-2) and between the connecting portion (213-3) and the stimulation portion (213-1).

4. The electrode array of claim 3, wherein each of the deformed portions (P1, P2) is formed to extend in the first direction.

5. The electrode array of claim 1, wherein each of the substrate (211) and the cover (214) has a first portion (217a) in which the plurality of electrodes (213) are disposed, and a second portion (217b), and a first cross-sectional area of the first portion (217a) is greater than a second cross-sectional area of the second portion (217b).

6. The electrode array of claim 1, wherein each of the plurality of electrodes includes platinum (Pt) and iridium (Ir).

7. A bioartificial implant system comprising:
a first unit (110); and
a second unit (200) configured to be communicatable with the first unit (100),
wherein the second unit (200) includes a circuit part (220) configured to generate a stimulation signal according to an electrical signal received from the first unit (100) and includes an electrode array (210) to which a current signal according to the stimulation signal is applied, wherein the electrode array (210) is any one of claims 1 to 6.

## Patentansprüche

1. Eine Elektrodenanordnung umfassend:
einen Körper (215) mit einer ersten Nut (H1), die sich in einer ersten Richtung (X) erstreckt;
ein Substrat (211), das auf der ersten Nut (H1) angeordnet ist;
eine Mehrzahl von Leitungen (212), die auf einer Oberfläche des Substrats (211) angeordnet sind;
mehrere Elektroden (213), die derart angeordnet sind, dass sie auf dem Substrat (211) in der ersten Richtung voneinander beabstandet sind, wobei jede der Vielzahl von Elektroden (213) einen Stimulationsabschnitt (213-1), einen ersten Endabschnitt (213-2) und einen Verbindungsabschnitt (213-3), der den Stimulationsabschnitt (213-1) mit dem ersten Endabschnitt (213-2) verbindet, umfasst und der erste Endabschnitt (213-2) elektrisch mit jeder der Mehrzahl von Leitungen (212) verbunden ist;
eine Abdeckung (214), die auf dem Substrat (211) angeordnet und derart ausgestaltet ist, dass sie den ersten Endabschnitt (213-2) jeder der mehreren Elektroden (213) abdeckt; und
ein Formteil (216), das derart ausgestaltet ist, dass es die Mehrzahl von Elektroden (213) an dem Körper (215) befestigt,
wobei der Stimulationsabschnitt (213-1) an einer Außenseite des Formteils freiliegt, und der erste Endabschnitt (213-2) den Stimulationsabschnitt (213-1) elektrisch mit der Vielzahl von Leitungen (212) über den Verbindungsabschnitt (213-3) verbindet,
wobei der Stimulationsabschnitt (213-1) tiefer angeordnet ist als die obere Oberfläche der ersten Nut (H1),
und
wobei einige Abschnitte der Abdeckung (214) und des Substrats (211) eingeschnitten sind, um Abschnitte der Mehrzahl von Elektroden (213) freizulegen.

2. Elektrodenanordnung nach Anspruch 1, wobei der Verbindungsabschnitt (213-3) vom ersten Endabschnitt (213-2) nach oben gebogen ist und der Stimulationsabschnitt (213-1) so angeordnet ist, dass er dem Substrat (211) vom Verbindungsabschnitt (213-3) zugewandt ist.

3. Elektrodenanordnung nach Anspruch 2, bei der durch teilweises Erhitzen verformte Abschnitte (P1, P2) zwischen dem Verbindungsabschnitt (213-3) und dem ersten Endabschnitt (213-2) und zwischen dem Verbindungsabschnitt (213-3) und dem Stimulationsabschnitt (213-1) vorhanden sind.

4. Elektrodenanordnung nach Anspruch 3, wobei jeder der verformten Abschnitte (P1, P2) so ausgebildet ist, dass er sich in der ersten Richtung erstreckt.

5. Elektrodenanordnung nach Anspruch 1, wobei sowohl das Substrat (211) als auch die Abdeckung (214) einen ersten Abschnitt (217a), in dem die mehreren Elektroden (213) angeordnet sind, und einen zweiten Abschnitt (217b) aufweist, und eine erste Querschnittsfläche des ersten Abschnitts (217a) größer ist als eine zweite Querschnittsfläche des zweiten Abschnitts (217b ).

6. Elektrodenanordnung nach Anspruch 1, wobei jede der mehreren Elektroden Platin (Pt) und Iridium (Ir) beinhaltet.

7. Ein bioartifizielles Implantatsystem, das Folgendes umfasst:
a) eine erste Einheit (110); und
b) eine zweite Einheit (200), die dazu eingerichtet ist, um mit der ersten Einheit (100) zu kommunizieren,
c) wobei die zweite Einheit (200) einen Schaltungsteil (220) enthält, der dazu eingerichtet ist, dass er ein Stimulationssignal entsprechend einem von der ersten Einheit (100) empfangenen elektrischen Signal zu erzeugen, und eine Elektrodenanordnung (210) enthält, an die ein Stromsignal entsprechend dem Stimulationssignal angelegt wird, wobei die Elektrodenanordnung (210) eine derjenigen der Ansprüche 1 bis 6 ist.

## Revendications

1. Réseau d'électrodes comprenant :
un corps (215) comprenant une première rainure (H1) s'étendant dans une première direction (X) ;
un substrat (211) disposé sur la première rainure (H1) ;
une pluralité de lignes (212) disposées sur une surface du substrat (211) ;
une pluralité d'électrodes (213) disposées de manière à être espacées les unes des autres sur le substrat (211) dans la première direction, chacune de la pluralité d'électrodes (213) comprenant une partie de stimulation (213-1), une première partie d'extrémité (213-2), et une partie de liaison (213-3) reliant la partie de stimulation (213-1) à la première partie d'extrémité (213-2), et la première partie d'extrémité (213-2) étant reliée électriquement à chacune de la pluralité de lignes (212) ;
un couvercle (214) disposé sur le substrat (211) et configuré pour couvrir la première partie d'extrémité (213-2) de chacune de la pluralité d'électrodes (213) ; et
un élément de moule (216) configuré pour fixer la pluralité d'électrodes (213) au corps (215),
dans lequel la partie de stimulation (213-1) est exposée à un côté externe de l'élément de moule, et la première partie d'extrémité (213-2) reliant électriquement la partie de stimulation (213-1) à la pluralité de lignes (212) via la partie de liaison (213-3),
dans lequel la partie de stimulation (213-1) est disposée plus bas que la surface supérieure de la première rainure (H1),
et dans lequel une partie du couvercle (214) et le substrat (211) sont incisés pour exposer des parties de la pluralité d'électrodes (213).

2. Réseau d'électrodes selon la revendication 1, dans lequel la partie de liaison (213-3) est pliée vers le haut à partir de la première partie d'extrémité (213-2) et la partie de stimulation (213-1) est disposée pour faire face au substrat (211) à partir de la partie de liaison (213-3).

3. Réseau d'électrodes selon la revendication 2, dans lequel des parties déformées (P1, P2) en étant partiellement chauffées sont incluses entre la partie de liaison (213-3) et la première partie d'extrémité (213-2) et entre la partie de liaison (213- 3) et la partie de stimulation (213-1).

4. Réseau d'électrodes selon la revendication 3, dans lequel chacune des parties déformées (P1, P2) est formée pour s'étendre dans la première direction.

5. Réseau d'électrodes selon la revendication 1, dans lequel chacun du substrat (211) et du couvercle (214) a une première partie (217a) dans laquelle la pluralité d'électrodes (213) est disposée, et une deuxième partie (217b), et une première superficie en coupe transversale de la première partie (217a) est supérieure à une deuxième superficie en coupe transversale de la deuxième partie (217b).

6. Réseau d'électrodes selon la revendication 1, dans lequel chacune de la pluralité d'électrodes comprend du platine (Pt) et de l'iridium (Ir).

7. Système d'implant bioartificiel comprenant :
une première unité (110) ; et
une deuxième unité (200) configurée pour pouvoir communiquer avec la première unité (100),
dans lequel la deuxième unité (200) comprend une partie de circuit (220) configurée pour générer un signal de stimulation selon un signal électrique reçu de la première unité (100) et comprend un réseau d'électrodes (210) auquel un signal de courant selon le signal de stimulation est appliqué, dans lequel le réseau d'électrodes (210) est l'un quelconque parmi les revendications 1 à 6.
